# EUROPEAN PATENT APPLICATION

(11) **EP 4 807 010 A1**
(43) Date of publication of application: **16.09.2026**
(21) Application number: 25163251.9
(22) Date of filing: 12.03.2025
(51) Int. Cl.: C12N 15/81, A61K 8/64, A61K 38/18, A61Q 19/00, C07K 14/49, C12N 1/16

(54) **LARGE SCALE PRODUCTION OF PLATELET-DERIVED GROWTH FACTOR PROTEIN**

(71) Applicant: Cogens S.r.l., 20841 Carate Brianza (IT)
(72) Inventor: CREMA, Giuliana, Albiate (IT); MANDELLI, Marialuisa, Brugherio (IT)
(74) Representative: Zaccaro, Elisabetta

(57) **Abstract**

The present invention concerns the field of recombinant protein production and in particular of Platelet-Derived Growth Factor (PDGF) production.

Specifically, the invention relates to a large-scale process for the preparation of toxin-free PDGF proteins, and in particular for the preparation of PDGF-BB. Further aspects of the invention relate to PDGF-BB protein obtainable by the process, and cosmetic and pharmaceutical uses of the protein.

## Description

### FIELD OF THE INVENTION

The present invention concerns the field of recombinant protein production and in particular of Platelet-Derived Growth Factor (PDGF) production.

Specifically, the invention relates to a large-scale process for the preparation of toxin-free PDGF proteins, and in particular for the preparation of PDGF-BB. Further aspects of the invention relate to PDGF-BB protein obtainable by the process, and cosmetic and pharmaceutical uses of the protein.

### STATE OF THE ART

Platelet-Derived Growth Factor (PDGF) plays a crucial role in various biological processes, including wound healing, angiogenesis, and tissue regeneration.

The increasing demand for PDGF in both therapeutic and research applications has driven the need for efficient production systems. While *Escherichia coli* (*E. coli*) is widely used for recombinant protein production, it presents several significant challenges, particularly when producing complex proteins like PDGF.

One of the primary issues with using *E. coli* for PDGF production is its inability to perform the necessary post-translational modifications (PTMs), such as glycosylation, phosphorylation, and disulfide bond formation. These modifications are essential for the proper folding, stability, and biological activity of PDGF. Because *E. coli* is a prokaryote, it lacks the sophisticated machinery required for these PTMs, which often results in an inactive or poorly functional protein that may not meet the therapeutic needs.

Another major flaw of using *E. coli* is the tendency for recombinant proteins like PDGF to form inclusion bodies. These are insoluble protein aggregates that occur when the protein folds improperly or becomes overwhelmed in the cellular environment. Inclusion bodies can significantly reduce the yield of active PDGF, as well as complicate the recovery process. To obtain functional PDGF, extensive and time-consuming refolding steps are often necessary, further reducing the overall efficiency of the production process.

Additionally, even when *E. coli* expresses PDGF successfully, the yields can be low compared to other expression systems. Purification of the protein also becomes challenging, as *E. coli* cultures often contain bacterial contaminants and endotoxins, which can compromise the purity of the final product. These contaminants can require expensive and labor-intensive purification processes, particularly for therapeutic applications where high purity is essential.

Finally, *E. coli* lacks the ability to efficiently secrete proteins into the culture medium, which limits its use for large-scale production of secreted proteins like PDGF. In contrast, many eukaryotic systems, such as yeast, insect, or mammalian cells, have natural secretion pathways that allow proteins to be more easily recovered from the culture medium, streamlining the downstream processing and reducing the overall cost and complexity of the production process.

While *E. coli* offers a cost-effective platform for many recombinant proteins, its limitations in terms of proper folding, post-translational modifications, inclusion body formation, and low yields make it less ideal for producing complex proteins like PDGF.

There are still very few cosmetic products/medical devices on the market that contain PDGF (Platelet-Derived Growth Factor) due to the complexity of its formulation:
- The production processes do not ensure a high degree of purification (not endotoxin-free).
- The protein is highly unstable when in contact with the epidermis.
- Effective formulations that ensure the protein reaches the dermis but not the systemic level (e.g., when applied to damaged skin to promote epithelial regeneration).

Incorporating PDGF into cosmetic or medical products is a challenging task because the processes needed to produce it can leave traces of endotoxins, which can trigger adverse reactions. Additionally, the protein's instability when exposed to the skin, especially the epidermis, means that it may not maintain its therapeutic effects.

Manufacturers need to carefully develop formulations that deliver PDGF to the right layer of the skin (the dermis) without allowing it to enter the bloodstream or cause systemic effects, especially when applied to injured or compromised skin. This ensures the product promotes localized healing without unintended side effects. There is an increasing need to explore alternative expression systems, that can overcome these challenges and offer a more efficient and reliable method for producing therapeutic-grade PDGF.

### SUMMARY OF THE INVENTION

PDGF, and in particular Platelet-Derived Growth Factor-BB (PDGF-BB) is a critical protein involved in cellular processes such as cell growth, migration, and wound healing. Given its importance, PDGF-BB holds substantial therapeutic potential, particularly in regenerative medicine, tissue engineering, and oncology.

The production of PDGF-BB, especially in large quantities with high purity and biological activity, has been a subject of extensive research and technological development.

The present invention discloses in a first aspect a process for a large-scale toxin-free Platelet-derived growth factor (PDGF) protein production comprising the steps of:
a. cloning a PDGF gene in an expression vector and transforming a *Pichia pastoris* host cell with said expression vector for producing the PDGF protein;
b. separating the host cells from the growth medium to isolate the PDGF protein;
c. spray-drying the isolated PDGF protein to obtain a powdered PDGF protein.

In a second aspect the present invention describes a purified PDGF protein, obtainable by the process of the invention, wherein said PDGF protein is PDGF-BB, wherein said protein is toxin-free. The platelet-derived growth factor (PDGF) family consists of four isoforms differing in the number of amino acid residues (PDGF-A 211 aa, PDGF-B 241 aa, PDGF-C 345 aa, PDGF-D 370 aa). The four isoforms share common structure, such as sequences involved in regulating the biological properties of these factors. The four different polypeptide chains can form homo- or heterodimers through the formation of disulfide bonds. The homodimer composed of two PDGF-B chains (PDGF-BB) is the most active isoform, as it is able to interact with and activate the three different isoforms of the PDGF receptor (PDGFRaa, PDGFRbb and PDGFRab).

The therapeutic application of protein-based growth factors is hindered by their short in vivo half-life and susceptibility to degradation.

A third aspect of the invention relates to a cosmetic composition or a medical device comprising a PDGF-BB protein obtainable by the process of the invention and at least one cosmetically acceptable carrier or excipient.

In a fourth aspect is described a pharmaceutical composition comprising a PDGF-BB protein obtainable by the present process and at least one pharmaceutically acceptable carrier or excipient.

A fifth aspect of the invention regards a cosmetic use of a PDGF-BB protein obtainable by the process herein described, or of the cosmetic composition.

In a sixth aspect it is herein described a PDGF-BB protein obtainable by the process or a pharmaceutical composition of the invention, for use as a medicament.

A seventh aspect of the invention relates to a PDGF-BB protein obtainable by the process of the present invention or a pharmaceutical composition or a medical device as herein described, for use in the treatment of wound healing and tissue regeneration.

### DETAILED DESCRIPTION OF THE INVENTION

The present invention concerns an efficient and reliable process for producing complex proteins such as therapeutic-grade PDGF. This alternative method allows to overcome the limitations in terms of proper folding, post-translational modifications, inclusion body formation, bacterial toxin contamination and low yields that occur with the commonly used platforms, such as those that make use of *E. coli* expression systems.

In a first aspect the present invention discloses a process for a large-scale toxin-free Platelet-derived growth factor (PDGF) protein production comprising the steps of:
a. cloning a PDGF gene in an expression vector and transforming a *Pichia pastoris* host cell with said expression vector for producing the PDGF protein;
b. separating the host cells from the growth medium to isolate the PDGF protein;
c. spray-drying the isolated PDGF protein to obtain a powdered PDGF protein. PDGF is one among numerous growth factors that regulate cell growth and division. In particular, PDGF plays a significant role in blood vessel formation, the growth of blood vessels from already-existing blood vessel tissue, proliferation of mesenchymal cells such as fibroblasts, osteoblasts, tenocytes, vascular smooth muscle cells and mesenchymal stem cells as well as chemotaxis.

PDGF is a dimeric glycoprotein that can be composed of two A subunits (PDGF-AA), two B subunits (PDGF-BB), or one of each (PDGF-AB).

In a preferred aspect of the process, the PDGF protein is the PDGF-BB protein.

In a further aspect, in the process according to the invention said step of producing the target gene protein in step a. is carried out at a temperature in the range from 21.6°C to 38.4°C, at a glucose concentration in the range from 0.3% to 3.7% and at a pH in the range from 5.3 to 8.7.

In a preferred aspect, in the process of the invention said producing the target gene protein in step a. is carried out at a temperature of 28.9°C, at a glucose concentration of 2.0% and at a pH of 6.9.

In a still more preferred aspect, in the process of the invention, step b. of separating the host cells from the growth medium to isolate PDGF protein is carried out by centrifugation or filtration.

In a second aspect the present invention describes a purified PDGF protein, obtainable by the process of the invention, wherein said a PDGF protein is PDGF-BB, wherein said protein is endotoxin-free. Besides being endotoxin-free, it also possesses favorable organoleptic properties for topical use. It has no unpleasant odor, eliminating the need for added fragrances in cosmetic creams or medical devices intended for application on damaged skin.

A third aspect of the invention relates to a cosmetic composition or a medical device comprising a PDGF-BB protein obtainable by the process of the invention and at least one cosmetically acceptable carrier or excipient.

In a fourth aspect is described a pharmaceutical composition comprising a PDGF-BB protein obtainable by the present process and at least one pharmaceutically acceptable carrier or excipient.

Preferably in the cosmetic composition or in the pharmaceutical composition or in the medical device, the PGDF-BB protein is encapsulated in a double-layer.

More preferably, the double layer is a PLGA polymer nanoparticle, further encapsulated within liposomes.

The PGDF-BB can be encapsulated. A particular PGDF-BB carrier configuration utilizes a double-layered encapsulation, in the first step, PDGF-BB is enclosed within stabilized PLGA polymer nanoparticles. These nanoparticles are then further encapsulated within liposomes to form the final delivery vehicle.

Encapsulating the PGDF-BB with this technology provides unexpected:
- high stability at pH of epidermis (stratus corneus), in fact the double layered encapsulation protect the PGDF-BB from endogenous proteases and allows the penetration of the capsule into the dermal layer and the delivery of PGDF-BB only at specific pH conditions.
- High homogeneity of particle size distribution (average diameter of particles is 140 nm and over 80% particles are below 100 nm in size)
- Efficient delivery of PGDF-BB into dermal layer by pH sensitive mechanism ensures that the hypodermis is not penetrated, thereby eliminating the risk of systemic absorption of the PGDF-BB.

Possible excipients are:
**Niacinamide:** A potent, cell-communicating ingredient that stimulates microcirculation in the dermis. This ingredient improves skin elasticity, enhances its barrier function, helps minimize discoloration and wrinkles, and revives the skin's healthy tone and texture. Niacinamide has a reputation for being able to treat uneven skin tone and mitigate acne and breakout.
**Tissue Inhibitor of Metalloproteinase-1 (rh-Polypeptide-58):** As a natural inhibitor of the matrix metalloproteinases, it decreases collagenase activity, thereby increasing collagen and elastin in the skin, while promoting cell proliferation and anti-apoptotic function.
**Sodium Hyaluronate:** The most effective humectant for skin healing and regeneration. It provides hydration to the skin without depending on atmospheric moisture.
**Vitamin B5:** Reduces water loss through the skin after microneedling. In various forms, topical vitamin B5 is beneficial in improving the moisture level and function of the skin.
**Magnesium Ascorbyl Phosphate (MAP):** A water-soluble vitamin C derivative that has better stability than L-ascorbic acid. MAP is often used for: UV protection and repair, collagen production,skin lightening and brightening, and as an antiinflammatory. It is also a potent antioxidant.
**Beta Glucan:** Stimulates immune defense, fibroblast growth, and collagen synthesis. It also improves the appearance of extrinsic signs of aging, while promoting healing and reducing scar discoloration.
**Lactoferrin:** antimicrobial and antifungal activity; it can scavenge free radicals, protecting cells from damage caused by oxidative stress. In some cases, lactoferrin can help regulate inflammation and reduce excessive immune responses.
**Sodium Chondroitin Sulfate:** improves skin hydration and elasticity.
**Ubiquinone (coenzyme Q10):** it is a potent antioxidant that neutralizes harmful free radicals, protecting cells from oxidative damage. It's particularly effective in protecting cell membranes and lipoproteins from lipid peroxidation, a process that can damage cells and contribute to various diseases.

Topical formulations are designed to meet specific needs in terms of texture, application, and the type of skin condition being treated. It is also very important that the formulations of the invention do not have persistent odors due to the PDGF production from bacteria. In fact the product obtained by the process of the invention, having the *Pichia pastoris* expression system, is odorless.

Topical formulations are applied directly to the skin or mucous membranes for local or systemic effects. They come in various forms, each designed to meet specific treatment needs or desired properties. The common types of topical formulations include:
**Creams:** Semi-solid emulsions of oil and water, often used to hydrate the skin or deliver active ingredients. Creams are versatile and can be used for a variety of skin conditions.
**Ointments:** Greasy, thick formulations that provide a barrier and are usually more occlusive than creams. Ointments are often used for dry, scaly skin conditions, or to protect the skin.
**Gels:** Clear or translucent semi-solid formulations that contain a gelling agent. Gels are typically non-greasy and are used for conditions where a cooling or drying effect is desired, such as acne or inflammation.
**Lotions:** Liquid formulations that are generally less thick than creams and are often used for moisturizing or for treating large areas of the skin. Lotions are easy to apply and absorb quickly.
**Solutions:** Liquid preparations of active ingredients dissolved in a solvent. Solutions are typically used for scalp treatments or areas where a thin, even application is necessary.
**Sprays:** Liquids or fine mists that are sprayed onto the skin for coverage. Sprays are often used for convenience or for covering larger areas quickly.
**Pastes:** Thick, solid formulations that contain a high proportion of solid material in a base, making them stiffer than creams or ointments. They are often used for protecting the skin or treating conditions like diaper rash or fungal infections.
**Emulsions:** Mixtures of water and oil, similar to creams but with varying water-to-oil ratios. Emulsions can provide different textures and absorption rates, depending on the specific formulation.
**Foams:** Aerated products that form a light, fluffy consistency upon application. Foams are often used for conditions that require easy spreadability or for sensitive areas.
**Patches:** Solid formulations that are adhered to the skin for continuous delivery of active ingredients. Patches can offer controlled-release drug delivery for pain management or hormone therapy.
**Tinctures:** Alcohol-based solutions that contain medicinal plant extracts or other active ingredients. Tinctures are often used for skin antiseptics or for treating fungal infections.
**Balms:** Thick, waxy preparations that offer soothing and moisturizing properties. Balms are often used for dry or chapped skin.

Each formulation type has unique characteristics that make it suitable for specific conditions, preferences, and purposes, depending on the desired effect and treatment area.

In a preferred aspect, the cosmetic or pharmaceutical compositions are for topical administration and are in the form of a cream, ointment, gel, lotion, solution, spray, paste, emulsion, foam, patch, tincture and balm. Preferably the topical composition is a cream.

A fifth aspect of the invention regards a cosmetic use of a PDGF-BB protein obtainable by the process herein described, or of the cosmetic composition.

In a sixth aspect it is herein described a PDGF-BB protein obtainable by the process or a pharmaceutical composition of the invention, for use as a medicament.

A seventh aspect of the invention relates to a PDGF-BB protein obtainable by the process of the present invention or a pharmaceutical composition or a medical device as herein described, for use in the treatment of wound healing and tissue regeneration.

*Pichia pastoris* protein expression systems offer many advantages, including ease of cultivation, fast growth, endotoxin-free protein production and scalability.

The production of PDGF-BB by the method of the present invention has evolved significantly through the development of advanced expression systems, optimized cultivation conditions, and refined purification techniques, achieving the status of gold standard for the production of biologically active PDGF-BB, especially for therapeutic applications.

The present process surprising allows to achieve advancements in scalability, cost-efficiency, and protein quality, enabling the broader use of PDGF-BB in medical and industrial settings.

Various embodiments and aspects of the present invention as delineated hereinabove and as claimed in the claims section below find experimental support in the following experimental section.

### EXPERIMENTAL SECTION

Reference is now made to the following experimental section, which together with the above descriptions illustrate some embodiments of the invention.

Large-scale production of PDGF-BB, as an example of the process which can be adapted to other proteins and in particular PDGF proteins, using the *Pichia pastoris* expression system.

### 1. Cloning of the PDGF-BB Gene

The process involves the design and synthesis of an optimized version of the PDGF-BB gene, specifically adapted for expression in *Pichia pastoris.* Once synthesized, this gene is inserted into an expression vector, which acts as a "vehicle" to transfer the gene into *Pichia* cells. After transformation, the cells that have successfully integrated the gene are selected, ensuring that only those capable of expressing the desired protein are used in the subsequent stages.

### 2. Heterologous Expression

In this phase, growth conditions are optimized for the yeast to maximize PDGF-BB production. Various culture and induction conditions are tested, as these factors stimulate the cells to produce the protein. Using deep well plates, different combinations of parameters such as culture medium composition and temperature are explored to identify the most efficient setup for production.

### 3. Biological and Chemical Characterization

Once PDGF-BB is produced, its quality is verified through a series of analyses. This phase involves both chemical and biological characterization of the product: the biological activity of PDGF-BB is measured, evaluating its effectiveness in terms of cellular stimulation, and its purity and concentration are assessed using chemical analysis methods. This ensures that the product meets the quality standards required by the cosmetic industry.

### 4. Initial Scale-Up

After optimizing the culture conditions on a small scale, the cells are grown in flasks, which are used during the initial growth phases. Once optimal conditions are determined, the culture is transferred to a 2-liter fermenter, representing an intermediate scale. During this phase, fermentation conditions are further explored, using specific culture media that promote PDGF-BB production and are particularly suitable for cosmetic use.

### 5. Downstream Processing

Following the production phase, the separation of cells from the culture broth is carried out using techniques such as centrifugation or filtration, which isolate the desired product. At this point, the PDGF-BB is converted into a powder through a spray drying process. This phase is crucial to preserve the integrity and biological activity of the protein, optimizing drying parameters to ensure a stable, high-quality product.

### 6. Scale-Up to a 100-Liter Fermenter

Once satisfactory results are achieved at the 2-liter fermenter level, the process moves to a larger scale, using a 100-liter fermenter. Here, controlling critical parameters such as pH, temperature, and dissolved oxygen concentration becomes even more important. Constant monitoring of these parameters ensures that the production process remains stable even at higher volumes.

### 7. Scale-Up of Downstream Processing

As the production capacity increases, the purification and spray drying processes are also optimized to handle larger volumes. The goal is to maintain the same product quality, regardless of batch size. This phase is essential for ensuring the scalability of the process, enabling its application at an industrial scale.

### 8. Pilot Batch Production

In this phase, three pilot batches of PDGF-BB are produced using the 100-liter fermenter. Pilot production is essential to verify the consistency of the product on a larger scale, with strict quality control throughout the process. Each batch is carefully monitored to ensure it meets the technical specifications established in the previous stages.

From the above description and the above-noted experimental section, the advantage attained by the process described and the product obtained according to the present invention are apparent.

## Claims

1. Process for a large-scale toxin-free Platelet-derived growth factor (PDGF) protein production comprising the steps of:
a. cloning a PDGF gene in an expression vector and transforming a *Pichia pastoris* host cell with said expression vector for producing the PDGF protein;
b. separating the host cells from the growth medium to isolate the PDGF protein;
c. spray-drying the isolated PDGF protein to obtain a powdered PDGF protein.

2. The process according to claim 1, wherein said PDGF protein is the PDGF-BB protein.

3. The process according to any one of claims 1 or 2, wherein said producing the target gene protein in step a. is carried out at a temperature in the range from 21.6°C to 38.4°C, at a glucose concentration in the range from 0.3% to 3.7% and at a pH in the range from 5.3 to 8.7.

4. The process according to any one of claims 1 to 3, wherein said producing the target gene protein in step a. is carried out at a temperature of 28.9°C, at a glucose concentration of 2.0% and at a pH of 6.9.

5. The process according to any one of claims 1 to 4, wherein step b. of separating the host cells from the growth medium to isolate PDGF protein is carried out by centrifugation or filtration.

6. A purified PDGF protein, obtainable by the process according to claims 1-5, wherein said a PDGF protein is PDGF-BB, wherein said protein is endotoxin-free.

7. A cosmetic composition comprising a PDGF-BB protein obtainable by the process according to claims 1-5 and at least one cosmetically acceptable carrier or excipient.

8. A pharmaceutical composition or a medical device comprising a PDGF-BB protein obtainable by the process according to claims 1-5 and at least one pharmaceutically acceptable carrier or excipient.

9. The cosmetic composition according to claim 7 or the pharmaceutical composition or the medical device according to claim 8, wherein the PGDF-BB protein is encapsulated in a double-layer.

10. The cosmetic composition or the pharmaceutical composition or the medical device according to claim 9, wherein said double layer is a PLGA polymer nanoparticle, further encapsulated within liposomes.

11. The cosmetic composition according to claim 7 or the pharmaceutical composition or the medical device according to claim 8, wherein said composition is for topical administration and is in the form of a cream, ointment, gel, lotion, solution, spray, paste, emulsion, foam, patch, tincture and balm.

12. A cosmetic use of a PDGF-BB protein obtainable by the process according to claims 1-5, or of the cosmetic composition according to claims 7 or 9-11.

13. A PDGF-BB protein obtainable by the process according to claims 1-5 or a pharmaceutical composition according to claims 8-11, for use as a medicament.

14. A PDGF-BB protein obtainable by the process according to claims 1-5 or a pharmaceutical composition according to claims 8-11, for use in the treatment of wound healing and tissue regeneration.
